# EUROPÄISCHE PATENTSCHRIFT

(11) **EP 3 188 788 B1**
(45) Veröffentlichungstag und Bekanntmachung des Hinweises auf die Patenterteilung: **27.09.2023**
(21) Anmeldenummer: 15749989.8
(22) Anmeldetag: 31.07.2015
(51) Int. Cl.: A61N 1/04, C23F 13/14

(54) **EMS-TRAININGSVORRICHTUNG, SOWIE EMS-KLEIDUNGSSTÜCK, UND VERFAHREN ZUR SICHERSTELLUNG DER FUNKTIONSFÄHIGKEIT EINER EMS-TRAININGSVORRICHTUNG**
EMS EXERCISE DEVICE, AS WELL AS EMS GARMENT AND METHOD FOR ENSURING THE FUNCTIONALITY OF AN EMS TRAINING DEVICE
DISPOSITIF D'ENTRAÎNEMENT EMS, AINSI QUE VÊTEMENT EMS ET PROCÉDÉ POUR GARANTIR LA FONCTIONNALITÉ D'UN DISPOSITIF D'ENTRAÎNEMENT EMS

(30) Priorität: 05.09.2014 DE 102014012920
(43) Veröffentlichungstag der Anmeldung: 12.07.2017
(73) Patentinhaber: Miha Bodytec GmbH, 86368 Gersthofen (DE)
(72) Erfinder: HORTER, Hansjürgen, 72644 Oberboihingen (DE); ÖSTERREICHER, Peter, 73249 Wernau (DE); DECKER, Jürgen, 86494 Emersacker (DE); PEINZE, Jonas, 90409 Nürnberg (DE)
(74) Vertreter: Pa-Munk
(86) Internationale Anmeldenummer: PCT/EP2015/025054
(87) Internationale Veröffentlichungsnummer: WO 2016/034291

(56) Entgegenhaltungen:
- CN-Y- 200 980 675
- DE-A1-102007 046 886
- US-A- 6 019 877
- US-A1- 2002 099 320
- US-B1- 6 845 272
- US-B1- 7 097 746

## Beschreibung

Die Erfindung betrifft eine EMS-Trainingsvorrichtung gemäß dem Oberbegriff des Anspruchs 9, sowie ein hierfür geeignetes EMS-Kleidungsstück gemäß dem Oberbegriff des Anspruchs 1 oder 4. Die Erfindung betrifft ferner ein Verfahren zum Betrieb einer solchen EMS-Trainingsvorrichtung gemäß dem Oberbegriff des Anspruchs 10.

Mit Elektromuskelstimulation (EMS), teilweise auch Elektromyostimulation genannt (EMS) werden Muskeln im lebenden Körper, im Regelfall zu Muskelaufbauzwecken, beispielsweise in Fitness-Studios oder bei Personal-Trainern mit elektrischen Reizen beaufschlagt, um die Muskeln zu kräftigen.

Dabei weisen übliche, am Körper anzulegende EMS-Elektroden häufig Textilstrukturen bzw. Textilstrukturabschnitte mit eingearbeiteten Metallfäden, insbesondere Silber- oder Kupfermetallfäden auf, oder Polymer-Pads, die mit leitfähigen Partikeln (z.B. Ruß) gefüllt sind. Eine EMS-Elektrode ist beispielsweise der deutschen Patentanmeldung DE 10 2007 046 886 A1 zu entnehmen.

Derartige EMS-Elektroden werden meistens vor dem Training genässt oder über einem genässten Unterbekleidungsstück, z.B. T-Shirt getragen, wozu sie in der Regel an einem am Körper anlegbaren Elektrodenträger angebracht sind. Während frühere Elektrodenträger häufig aus Riemen- und Lederbändern bestanden, an denen die Elektroden angebracht waren, geht die Entwicklung immer mehr hin zu EMS-Kleidungsstücken, insbesondere textilen Kleidungsstücken, die die Elektroden tragen und selbst von der trainierenden Person eben wie ein Kleidungsstück getragen werden können, also beispielsweise als Weste, Hose, Strumpf, Armband oder dergleichen. Um die Akzeptanz der Elektromuskelstimulation (EMS) dabei weiter zu erhöhen, wird versucht, die Elektroden und die an die Elektroden angeschlossenen Leiterbahnen immer besser in das EMS-Kleidungsstück zu integrieren, um so die sichtbaren Kabel zu eliminieren oder zumindest auf die von dem EMS-Kleidungsstück zu einer externen Steuereinheit führenden Kabel zu verkürzen, sowie die Elektroden nicht nur weitestgehend unsichtbar zu machen, sondern auch den Tragekomfort des EMS-Kleidungsstücks allgemein zu erhöhen, in dem die EMS-Elektroden möglichst flexibel und dehnbar, häufig aus textilen Materialien, gebildet werden. Ein Beispiel für ein solches EMS-Kleidungsstück ist dem deutschen Patent DE 10 2009 017 179 B4 zu entnehmen. Ein weiteres EMS-Kleidungsstück mit aufgeklebten, textilen EMS-Elektroden zeigt die Gebrauchsmusterschrift DE 20 2011 050 682 U1.

Zwar gibt es heute schon akkumulatorbetriebene EMS-Reizerzeugungseinheiten, welche die über die EMS-Elektroden auf den Körper abzugebenden EMS-Reize formen und somit direkt am EMS-Kleidungsstück und damit am Körper "autark" getragen werden können, siehe DE 20 2011 109 226 U1, WO 2011/089 263 A2 und WO 2014/000 736 A2 . In der Regel reicht die Akkuleistung jedoch nicht, um über einen im Trainingsbetrieb akzeptablen Zeitraum diese EMS-Reize zu erzeugen, welche aus Stromimpulsen und/oder Wechselstrom bestehen. Zu der Erzeugung dieser EMS-Reize weist die EMS-Reizerzeugungseinrichtung in der Regel einen elektrischen Impulsgenerator auf, sowie eine elektronische Steuerung, welche ein Anregungsschema vorgibt, dem folgend aus einem von einer Stromquelle bezogenen Strom eine zeitlich und auf die EMS-Elektroden des EMS-Trainingsgerätes verteilte Mehrzahl von EMS-Reizen geformt wird, die aus den Stromimpulsen und/oder Wechselstrom mit per Steuerung vorgegebenen Werten wie Amplitude und Frequenz bestehen, und mit denen die EMS-Elektroden beaufschlagt werden, um durch den Körper Strom mit vorgegebener Amplitude und Frequenzmuster durch zu leiten. Dabei sind die EMS-Elektroden an dem EMS-Kleidungsstück üblicherweise in Paaren gruppiert, so dass zwei zu einem solchen Paar gruppierte EMS-Elektroden jeweils über einen Leitungsast an der EMS-Reizerzeugungseinheit angeschlossen sind, die während des EMS-Trainings durch den Körper, an dem sie aufliegen, zu einem geschlossenen Stromkreis vervollständigt werden, der durch den Körper und somit durch die Muskeln hindurch führt.

Dieser Stromfluss kann bei der Verwendung von mehr als einem Elektrodenpaar natürlich wesentlich komplexer sein und Stromflüsse zwischen mehr als zwei verschiedenen EMS-Elektroden beinhalten. Ferner wird üblicherweise unter dem EMS-Kleidungsstück bzw. dem Elektrodenträger mit den EMS-Elektroden ein genässtes Kleidungsstück getragen, oft beispielsweise ein handelsübliches T-Shirt, wobei mittlerweile auch EMS-Unterbekleidung erhältlich ist, die zur Durchleitung der die EMS-Reize bildenden Ströme und auf Saugfähigkeit optimiert ist und entsprechend ebenfalls im Stromkreis zwischengeschaltet ist. Dabei werden im Regelfall die EMS-Elektroden paarweise so geschaltet, dass bei einem Zeitpunkt die eine EMS-Elektrode mit einem Impulsstrom beaufschlagt wird und die andere zu diesem Zeitpunkt nicht, wobei die beiden zum Körper führenden Leitungsäste mit dem zum Nässen der EMS-Elektroden üblicherweise verwendeten Wasser und dem Schweiß auf der Hautoberfläche in Kontakt kommen. Beim Impulswechsel wird dann die andere EMS-Elektrode mit Strom impulsartig beaufschlagt und die erste Elektrode nicht.

Wie beispielsweise der deutschen Patentanmeldung DE 102 48 235 A1 entnommen werden kann sind die die EMS-Elektroden mit der EMS-Reizerzeugungseinheit verbindenden Leitungsäste dabei zumindest zwischen einer Anschlussstelle am EMS-Kleidungsstück und der EMS-Reizerzeugungseinheit als EMS-Signalkabel ausgebildet, die von einer üblicherweise entfernt von dem Trainierenden und somit von den getragenen EMS-Kleidungsstücken angeordneten EMS-Reizerzeugungseinheit direkt zu den EMS-Elektroden oder zu entsprechenden Anschlüssen an den bzw. dem EMS-Kleidungsstück führen. Die EMS- Reizerzeugungseinheit ist dabei üblicherweise in einer eine Bedienoberfläche aufweisenden Steuereinheit, häufig in Form eines Steuerpults, untergebracht und an das reguläre Stromnetz angeschlossen. Eine solche Steuereinheit ist prinzipiell, wenngleich auf dem technischen Stand von Anfang der 90er Jahre, der europäischen Patentschrift EP 0 459 945 B1 zu entnehmen. Solche EMS-Trainingsgeräte werden von verschiedenen Herstellern produziert und professionell in Fitnessstudios von Personal-Trainern etc. betrieben, aber auch von Privatpersonen direkt.

Während die Erfolge der EMS-Methode beim Muskelaufbau unbestritten sind und EMS-Trainingsgeräte beim Publikum aufgrund der immer mehr verbesserten äußeren Erscheinung auch auf immer größeres Interesse stoßen, treten aufgrund des vermehrten Einsatzes auch systemische Probleme deutlicher zu Tage. So kommt es im Gebrauch zu unerklärlichen und zeitlich zufällig auftretenden Ausfällen von EMS-Elektroden, was bei den heutigen EMS-Kleidungsstücken, in die die Elektroden bereits integriert sind, zum Austausch des gesamten EMS-Kleidungsstücks, also beispielsweise einer Trainingsweste führt. Elektrodenausfall bedeutet in diesem Zusammenhang, dass die betroffene EMS-Elektrode keinen oder keinen ausreichenden Impulsstrom mehr an den Körper abgibt, obwohl keine Stromleitung oder Kontaktstelle zwischen der EMS-Elektrode und der die EMS-Elektrode mit Strom beaufschlagenden EMS-Reizerzeugungseinheit gebrochen und auch an der Elektrode äußerlich kein Defekt zu sehen sein muss.

Hiervon ausgehend ist es Aufgabe der vorliegenden Erfindung, ein EMS-Kleidungsstück, sowie eine EMS-Trainingsvorrichtung und ein Verfahren zum Betrieb einer EMS-Trainingsvorrichtung zu schaffen, mit denen die Funktionsfähigkeit der in einer EMS-Trainingsvorrichtung eingesetzten EMS-Elektroden bzw. EMS-Kleidungsstücke über einen langen Zeitraum sichergestellt werden kann.

Diese Aufgabe wird hinsichtlich des EMS-Kleidungsstücks mit den Merkmalen des Anspruchs 1 oder 4 gelöst, hinsichtlich der EMS-Trainingsvorrichtung mit den Merkmalen des Anspruchs 9 und hinsichtlich des Verfahrens zum Betrieb einer EMS-Trainingsvorrichtung mit den Merkmalen des Anspruchs 10.

Ein Verfahren ist offenbart, bei dem in den Stromkreis, der von der EMS-Reizerzeugungseinheit über ein EMS-Signalkabel und eine EMS-Elektrode, den auf der Hautoberfläche des Trainierenden befindlichen Schweiß, die Hautoberfläche selbst und das Körperinnere und wieder aus dem Körperinneren durch die Haut und den auf der Haut befindlichen Schweiß hindurch zu einer weiteren EMS-Elektrode und von dort über eine entsprechende EMS-Signalleitung zurück zur EMS-Reizerzeugungseinheit geschlossen ist, eine, bevorzugt auf beiden Seiten des Körpers eine, Opferanode eingebracht wird. Dazu wird zumindest eine der EMS-Elektroden vor dem nach Abschluss des Verfahrens durchführbaren EMS-Training mit einer Flüssigkeit genässt und/oder einem Gel bestrichen, in der bzw. in dem Teilchen enthalten, insbesondere gelöst sind, welche als die Opferanode wirken und dazu aus dem Material mit negativem Standardelektrodenpotential in der elektrochemischen Spannungsreihe bestehen oder ein solches aufweisen, insbesondere aus dem Magnesium oder aus Magnesiumlegierungen, welches frei von Buntmetall wie z.B. Nickel ist.

Erfindungsgemäß wird ferner eine EMS-Trainingsvorrichtung mit den Merkmalen des Anspruchs 9 vorgeschlagen, bei der die Opferanode(n) in diesen Stromkreis eingebracht ist bzw. sind. Erfindungsgemäß werden ferner Hilfsmittel vorgeschlagen, um die Opferanode(n) dort einzubringen, nämlich ein die Opferanode(n) aufweisendes EMS-Kleidungsstück mit den Merkmalen des Anspruchs 1 oder 4.

Der Erfindung liegt dabei die Erkenntnis zugrunde, dass unterschiedliche, auch temporär unterschiedliche Widerstände in dem von der EMS-Reizerzeugungseinheit über die erste EMS-Elektrode zum Körper führenden Leitungsast im Vergleich zu dem über die zweite EMS-Elektrode zurück zur EMS-Reizerzeugungseinheit führenden Leitungsast auftreten können. Diese Widerstände werden durch sich zeitlich ändernde Gegebenheiten im Stromkreis, wie z.B. der unterschiedlich guten Anlage der jeweiligen EMS-Elektrode am Körper, lokal unterschiedlich starker Schweißabsonderung an den Hautoberflächen, unterschiedlich starker Nässung der beiden EMS-Elektroden etc. hervorgerufen, aber auch durch systemische Fehler, beispielsweise an den Verbindungsstellen der EMS-Elektroden mit EMS-Signalverbindungskabeln, die häufig als Druckknöpfe oder Crimp-Stecker ausgebildet sind. Durch diese Widerstandsunterschiede kommt es dann zu einer Oxidation und damit Korrosion an unterschiedlichen Stellen im Stromkreis, wobei an den Korrosionsstellen unter Elektronenabgabe das dort vorhandene metallische Material oxidiert und damit korrodiert.

Dadurch wird an den korrodierenden Stellen in elektrischer Hinsicht der Aufbau eines Zwischenwiderstands bewirkt, der über die Zeit soweit gehen kann, dass die im betroffenen Leitungsast angeordnete EMS-Elektrode keinen für die gewünschte Muskelkontraktion nötigen EMS-Reiz mehr übertragen kann.

Ist dieser Leitungsast mit einer Opferanode elektrisch leitend verbunden, so korrodiert an Stelle der EMS-Elektrode, einer Verbindungsstelle von EMS-Elektrode und EMS-Signalkabel oder anderer anfälliger Stellen im Leitungsast die Opferanode, d.h. sie opfert sich selbst, bis sie vollständig korrodiert ist. Dadurch lässt sich die Haltbarkeit der EMS-Ausrüstung, d.h. der EMS-Elektroden bzw. des teuren EMS-Kleidungsstücks, welches als Träger für die EMS-Elektroden fungiert, soweit verlängern, bis diese ihre wirtschaftliche Nutzungsdauer aufgrund sonstiger üblicher Abnutzung beim Training erreicht haben.

Dabei ist vorteilhaft jeder EMS-Elektrode bzw. jedem von der EMS-Reizerzeugungseinheit zur jeweiligen EMS-Elektrode eine eigene Opferanode zugeordnet, d.h. die Opferanode ist mit dem jeweiligen Leitungsast elektrisch leitend verbunden. Es wäre jedoch auch denkbar, eine gemeinsame Opferanode für mehrere Leitungsäste vorzusehen und mit mehreren Leitungsästen leitend zu verbinden, beispielsweise mit solchen, die gleichgetaktet mit EMS-Reizen, also beispielsweise Stromimpulsen beschickt werden, oder sogar - mit entsprechender Verkabelung bzw. entsprechender Verschaltung - sternförmig mit allen Leitungsästen. Möglicherweise wäre es aufgrund der komplexen Stromflüsse bei einer heutigen EMS-Trainingsvorrichtung mit einer Vielzahl von EMS-Elektroden sogar möglich, den vorstehend beschriebenen Korrosionsschutzeffekt zu erzielen, ohne dass alle Leitungsäste selbst direkt mit einer Opferanode in Verbindung stehen.

Geeignete Stellen zum Anbringen der Opferanode(n) sind die Druckknöpfe der EMS-Elektroden oder der elektrischen Anschlüsse des EMS-Kleidungsstücks, an denen Litzen der Stromkabel am EMS-Kleidungsstück bzw. an der EMS-Elektrode üblicherweise durch Anlöten, Annähen oder Einklemmen zwischen den beiden Teilen des Druckknopfs befestigt werden und/oder ein eine Leitschicht der EMS-Elektrode bildendes textiles Gewebe, Gewirk oder Gestrick mit metallischen oder metallisierten Fäden (beispielsweise Silberfäden oder silberhaltige Fäden). Dort ist daher erfindungsgemäß eine als Metallplättchen oder -streifen geformte Opferanode angebracht, z.B. einfach aufgeklebt, wobei sich der elektrische Anschluss an dem jeweiligen Leitungsast auf einfache Weise kostengünstig herstellen lässt.

Eine weitere schnelle und einfache Befestigung der Opferanode wäre das Aufnieten auf den Druckknopf am EMS-Kleidungsstück bzw. an der EMS-Elektrode. Dabei kann die plättchenförmige Opferanode mittels einer oder mehrerer anderer Nieten aufgenietet werden oder selbst als Niet ausformt und aufgenietet sein.

Gemäß einer ersten Alternative des erfindungsgemäßen Verfahrens wird die EMS-Elektrode dagegen mit einem Gel bestrichen, welches Opferanodeneigenschaften aufweist und somit die Opferanode bildet. Eine weitere erfindungsgemäße Möglichkeit, eine Opferanode in den von der EMS-Reizerzeugungseinheit über die beiden einander zugeordneten EMS-Elektroden und zurück zur EMS-Reizerzeugungseinheit geschlossenen Stromkreis oder in jeden der beiden Leitungsäste einzubringen besteht darin, die zum Nässen der EMS-Elektroden verwendete Flüssigkeit - alternativ oder ergänzend zu den vorstehend beschriebenen Festkörperopferanoden - mit Teilchen zu versehen, die die gewünschte Opferanodeneigenschaft eines negativen Standardelektrodenpotentials in der elektrochemischen Spannungsreihe aufweisen, und dann anstatt des Leitermetalls in den EMS-Elektroden bzw. deren Zuleitungen oxydieren und per Schweiß nach dem Training abtransportiert werden oder in einem unter dem EMS-Elektroden in den meisten Fällen getragenen Unterbekleidungsstück zurück bleiben.

Die Erfindung betrifft somit ein EMS-Kleidungsstück mit den Merkmalen des Anspruchs 1 oder 4, wie z.B. Anzug, Jacke, Gürtel, Shirt, Hose, Arm- und/oder Beinband, welches eine erste Anzahl, insbesondere Mehrzahl EMS-Elektroden trägt, und insbesondere eine Textilie ist, wobei die erste Anzahl, insbesondere Mehrzahl EMS-Elektroden zur Übertragung von aus Stromimpulsen und/oder Wechselstrom mit vorgegebenen Werten wie Amplitude und Frequenz bestehenden EMS-Reizen von einer angeschlossenen EMS-Reizerzeugungseinheit auf den lebenden Körper jeweils als ein sich flexibel an den Körper anlegendes, flächiges Pad oder als ein Textilstrukturabschnitt mit einer elektrisch leitenden Leitschicht ausgebildet sind, insbesondere mit einer metallische oder metallisierte Fäden enthaltenden textilen Leitschicht. Das erfindungsgemäße EMS-Kleidungsstück weist zumindest eine, vorzugsweise für jede EMS-Elektrode eine eigene Opferanode auf, welche mit der jeweiligen EMS-Elektrode elektrisch leitend in Verbindung steht, wobei die Opferanode aus einem Material, vorzugsweise einem Metall mit einem negativen Standardelektrodenpotential in der elektrochemischen Spannungsreihe besteht oder ein solches aufweist, insbesondere Magnesium oder Magnesiumlegierungen, aber vorteilhaft frei von Buntmetall wie z.B. Nickel ist.

Die Erfindung betrifft ferner eine EMS-Trainingsvorrichtung, mit einer dritten Anzahl, insbesondere Mehrzahl am lebenden Körper insbesondere paarweise einander zugeordnet anbringbarer EMS-Elektroden, zur Beaufschlagung des Körpers mit EMS-Reizen, einer vierten Anzahl mit der dritten Anzahl EMS-Elektroden elektrisch leitend verbundener EMS-Reizerzeugungseinheiten, welche aus einem von einer Stromquelle bezogenen Strom eine einem vorgegebenen Anregungsschema folgende, zeitlich und elektrodenmäßig verteilte Mehrzahl von EMS-Reizen formt, die aus Stromimpulsen und/oder Wechselstrom mit vorgegebenen Werten wie Amplitude und Frequenz bestehen, und mit der die dritte Anzahl EMS-Elektroden beaufschlagt werden, um durch den Körper Strom mit vorgegebener Amplitude und Frequenzmuster durchzuleiten, insbesondere zwischen paarweise einander zugeordneten EMS-Elektroden. Die erfindungsgemäße EMS-Trainingsvorrichtung zeichnet sich dadurch aus, dass die EMS-Trainingsvorrichtung zum Schutz der dritten Anzahl EMS-Elektroden und/oder anderer Elemente in den elektrisch leitenden Verbindungen vor Korrosion zumindest eine, vorzugsweise für jede EMS-Elektrode eine eigene, elektrisch leitend mit der EMS-Elektrode in Verbindung stehende Opferanode aufweist, sowie zumindest ein EMS-Kleidungsstück mit den Merkmalen des Anspruchs 1 oder 4, wobei jede Opferanode aus einem Material, vorzugsweise einem Metall mit einem negativen Standardelektrodenpotential in der elektrochemischen Spannungsreihe besteht oder ein solches aufweist, insbesondere Magnesium oder Magnesiumlegierungen, aber vorteilhaft frei von Buntmetall wie z.B. Nickel ist, und wobei jede Opferanode vorzugsweise als Metallplättchen, -streifen, oder -formteil ausgeformt ist.

Vorteilhafte Weiterbildungen dieser Gegenstände sind Gegenstand der den nebengeordneten Hauptansprüchen jeweils zugeordneten Unteransprüche.

In den beiliegenden Figuren werden verschiedene Ausführungsformen in der Erfindung näher erläutert. Es zeigen:
- Figur 1: eine schematische Ansicht einer EMS-Trainingsvorrichtung gemäß einer ersten Ausführungsform der Erfindung;
- Figur 2: eine Einzelheit aus Figur 1 in vergrößerter Darstellung;
- Figur 3: ein EMS-Signalkabel, jedoch nicht gemäß einer Ausführungsform der Erfindung; und
- Figur 4: eine schematische Darstellung einer EMS-Reizerzeugungseinheit, jedoch ebenfalls nicht gemäß einer Ausführungsform der Erfindung, mit einem zugeordneten EMS-Signalkabel.

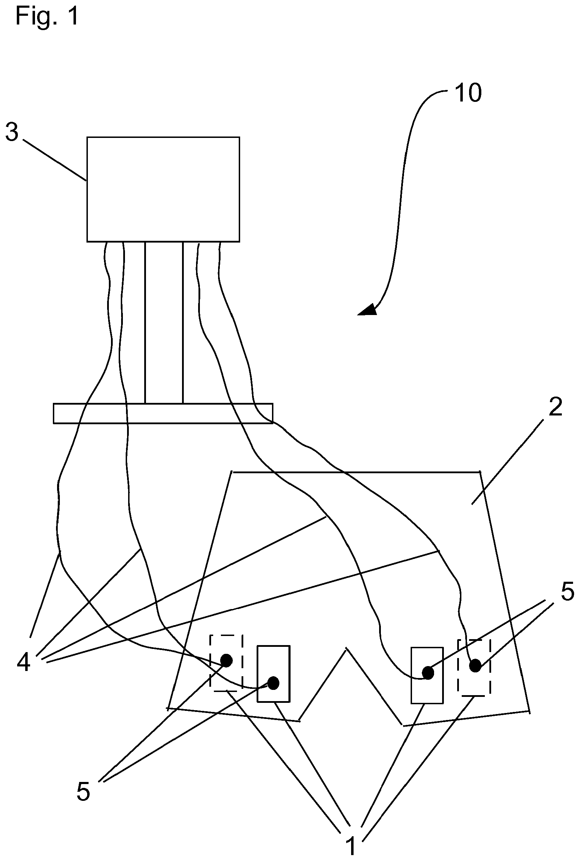

In der Figur 1 ist ein als kurze Hose gestaltetes EMS-Kleidungsstück 2 gezeigt, welches mit vier jeweils zu zwei Paaren gruppierten EMS-Elektroden 1 bestückt ist, wobei jedes Elektrodenpaar einem Oberschenkel zugeordnet ist, und wobei jede EMS-Elektrode 1 über ein EMS-Signalkabel 4 mit einer in ein pultförmiges Steuergerät 3 eingebauter EMS-Reizerzeugungseinheit verbunden ist. Die EMS-Elektroden 1 weisen dabei Druckknöpfe 5 auf, an denen die EMS-Signalkabel 4 mit entsprechenden Steckern aufgesteckt sind.

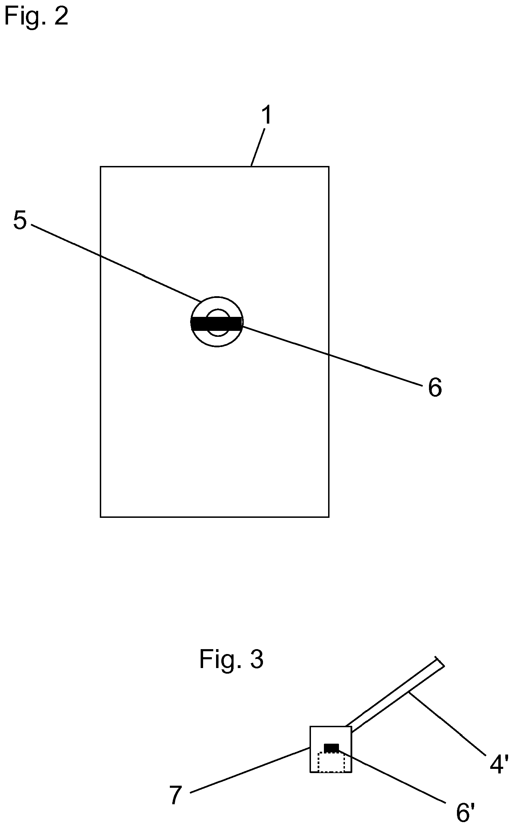

Figur 2 zeigt eine der EMS-Elektroden 1 von der Innenseite des Hosenbeins aus gesehen, wobei eine dem Körper zugewandte elektrische Leitschicht abgenommen worden ist, so das man die Rückseite des Druckknopfs 5 erkennt, an der eine Opferanode 6 in Form eines Magnesiumstreifens aufgeklebt ist.

Ergänzend kann anstatt eines Standard-EMS-Signalkabels 4 ein in Figur 3 gezeigtes EMS-Signalkabel 4' eingesetzt werden, welches mit einer Opferanode 6' versehen ist. In der dargestellten Ausführungsform ist die Opferanode 6' dabei in einen Stecker 7 integriert, welcher auf einen der Druckknöpfe 5 oder einen entsprechenden Druckknopf einer EMS-Elektrode aufgesteckt werden kann, um die EMS-Elektrode der EMS-Reizerzeugungseinheit 3 zu verbinden.

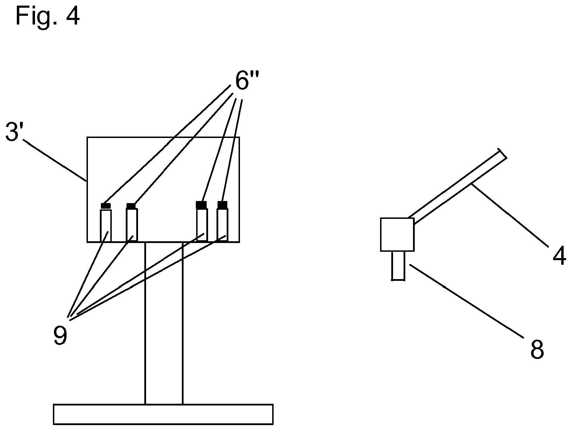

Ergänzend kann eine in Figur 4 schematisch dargestellte EMS-Reizerzeugungseinheit 3' vorgesehen sein, welche ebenfalls in einer schaltpultartigen äußeren Gestalt verbaut ist, aber im Gegensatz zu der in Figur 1 gezeigten EMS-Reizerzeugungseinheit 3 für jedes der EMS-Signalkabel bzw. dessen mit einem Stecker 8 versehenen Ende eine Steckdose 9 aufweist, in der eine Opferanode 6" angeordnet, vorzugsweise austauschbar angeordnet ist.

Abwandlungen und Modifikationen der gezeigten Ausführungsformen sind möglich, ohne den Rahmen der Erfindung zu verlassen.

## Patentansprüche

1. EMS-Kleidungsstück (2), wie z.B. Anzug, Jacke, Gürtel, Shirt, Hose, Arm- und/oder Beinband, welches eine erste Anzahl EMS-Elektroden (1) trägt, wobei die erste Anzahl EMS-Elektroden (1) zur Übertragung von aus Stromimpulsen und/oder Wechselstrom mit vorgegebenen Werten wie Amplitude und Frequenz bestehenden EMS-Reizen von einer angeschlossenen EMS-Reizerzeugungseinheit (3) auf den lebenden Körper jeweils als ein sich flexibel an den Körper anlegendes, flächiges Pad oder als ein Textilstrukturabschnitt mit einer elektrisch leitenden, metallische oder metallisierte Fäden enthaltenden, textilen Leitschicht ausgebildet sind, **dadurch gekennzeichnet, dass** das EMS-Kleidungsstück (2) zumindest eine Opferanode (6) aufweist, welche mit der jeweiligen EMS-Elektrode (1) elektrisch leitend in Verbindung steht, wobei die Opferanode (6) aus einem Material mit einem negativen Standardelektrodenpotential in der elektrochemischen Spannungsreihe besteht oder ein solches aufweist, wobei die zumindest eine eine Opferanode (6) tragende EMS-Elektrode (1) einen Crimpstecker, einen Druckknopf (5) oder eine Pressverbindungstülle aufweist, an dem bzw. der die Leitschicht elektrisch leitend eingepresst, damit verschweißt oder vernäht ist, und/oder an dem bzw. der eine EMS-Signalleitung eingeklemmt, angelötet, angenäht oder mit einem passenden Stecker auf- bzw. eingesteckt ist, wobei die Opferanode (6) als an dem Crimpstecker, Druckknopf (5) oder der Pressverbindungstülle angebrachtes Metallplättchen oder -streifen ausgeformt ist.

2. EMS-Kleidungsstück (2) nach Anspruch 1, **dadurch gekennzeichnet, dass** das EMS-Kleidungsstück (2) zumindest eine eine Opferanode (6) tragende EMS-Elektrode (1) aufweist, welche mit der Leitschicht der EMS-Elektrode (1) elektrisch leitend verbunden oder verbindbar ist.

3. EMS-Kleidungsstück (2) nach Anspruch 1 oder 2, **dadurch gekennzeichnet, dass** die zumindest eine eine Opferanode (6) tragende EMS-Elektrode (1) eine aufgenähte EMS-Elektrode (1) ist und/oder eine in einem textilen Flächenherstellungsverfahren wie z.B. Wirken, Stricken, Weben, Vliesherstellung eingearbeitete EMS-Elektrode.

4. EMS-Kleidungsstück (2), wie z.B. Anzug, Jacke, Gürtel, Shirt, Hose, Arm- und/oder Beinband, welches eine erste Anzahl EMS-Elektroden (1) trägt, wobei die erste Anzahl EMS-Elektroden (1) zur Übertragung von aus Stromimpulsen und/oder Wechselstrom mit vorgegebenen Werten wie Amplitude und Frequenz bestehenden EMS-Reizen von einer angeschlossenen EMS-Reizerzeugungseinheit (3) auf den lebenden Körper jeweils als ein sich flexibel an den Körper anlegendes, flächiges Pad oder als ein Textilstrukturabschnitt mit einer elektrisch leitenden, metallische oder metallisierte Fäden enthaltenden, textilen Leitschicht ausgebildet sind, **dadurch gekennzeichnet, dass** das EMS-Kleidungsstück (2) zumindest eine Opferanode (6) aufweist, welche mit der jeweiligen EMS-Elektrode (1) elektrisch leitend in Verbindung steht, wobei die Opferanode (6) aus einem Material mit einem negativen Standardelektrodenpotential in der elektrochemischen Spannungsreihe besteht oder ein solches aufweist, wobei das EMS-Kleidungsstück eine zweite Anzahl elektrischer Anschlüsse für die erste Anzahl EMS-Elektroden aufweist, welche zweite Anzahl elektrischer Anschlüsse jeweils als Crimpstecker, Druckknopf oder Pressverbindungstülle gestaltet ist, und welche über darin eingepresste oder damit vernähte, verlötete oder verschweißte EMS-Signalleitungen mit der ersten Anzahl EMS-Elektroden verbunden sind, wobei an der zweiten Anzahl Anschlüsse EMS-Signalkabel zur Verbindung mit der EMS-Reizerzeugungseinheit anschließbar sind, wobei an den Anschlüssen jeweils eine Opferanode elektrisch leitverbunden angebracht ist.

5. EMS-Kleidungsstück nach Anspruch 4, **dadurch gekennzeichnet, dass** die die zweite Anzahl elektrischer Anschlüsse und die erste Anzahl EMS-Elektroden verbindenden EMS-Signalleitungen als in das EMS-Kleidungsstück integrierte, beispielsweise eingestrickte oder eingewobene Leiterbahnen ausgebildet sind.

6. EMS-Kleidungsstück nach Anspruch 1 oder 4, **dadurch gekennzeichnet, dass** die Opferanode als Metallplättchen oder -streifen geformt und auf den Druckknopf der EMS-Elektrode oder des Anschlusses aufgeklebt ist.

7. EMS-Kleidungsstück nach Anspruch 2 oder 3, **dadurch gekennzeichnet, dass** die Opferanoden an den Anschlüssen jeweils textil montiert wie z.B. aufgestickt oder aufgenäht sind.

8. EMS-Kleidungsstück nach einem der vorhergehenden Ansprüche, **dadurch gekennzeichnet, dass** das Material mit einem negativen Standardelektrodenpotential in der elektrochemischen Spannungsreihe, aus dem die Opferanode (6) besteht oder das die Opferanode (6) aufweist, Magnesium oder eine Magnesiumlegierung ist, wobei die Opferanode (6) frei von Buntmetall wie z.B. Nickel ist.

9. EMS-Trainingsvorrichtung (10), mit einer dritten Anzahl am lebenden Körper anbringbarer EMS-Elektroden (1), zur Beaufschlagung des Körpers mit EMS-Reizen,
einer vierten Anzahl mit der dritten Anzahl EMS-Elektroden (1) elektrisch leitend verbundener EMS-Reizerzeugungseinheiten (3), welche aus einem von einer Stromquelle bezogenen Strom eine einem vorgegebenen Anregungsschema folgende, zeitlich und elektrodenmäßig verteilte Mehrzahl von EMS-Reizen formt, die aus Stromimpulsen und/oder Wechselstrom mit vorgegebenen Werten wie Amplitude und Frequenz bestehen, und mit der die dritte Anzahl EMS-Elektroden (1) beaufschlagt wird, um durch den Körper Strom mit vorgegebener Amplitude und Frequenzmuster durchzuleiten,
**dadurch gekennzeichnet, dass**
die EMS-Trainingsvorrichtung (10) zum Schutz der dritten Anzahl EMS-Elektroden (1) und/oder anderer Elemente in den elektrisch leitenden Verbindungen vor Korrosion zumindest eine elektrisch leitend mit der EMS-Elektrode (1) in Verbindung stehende Opferanode (6) aufweist, wobei
jede Opferanode (6) aus einem Material mit einem negativen Standardelektrodenpotential in der elektrochemischen Spannungsreihe besteht oder ein solches aufweist, wobei
die EMS-Trainingsvorrichtung (10) zumindest ein EMS-Kleidungsstück (2) nach einem der Ansprüche 1 bis 8 aufweist.

10. Verfahren zur Sicherstellung der Funktionsfähigkeit von in einer EMS-Trainingsvorrichtung nach Anspruch 9 eingesetzten EMS-Elektroden bzw. EMS-Kleidungsstücken nach Anspruch 1 bis 8 über einen langen Zeitraum, **dadurch gekennzeichnet, dass** auf einer oder auf beiden Seiten des Körpers eine Opferanode eingebracht wird, indem
zumindest eine der EMS-Elektroden vor dem nach Abschluss des Verfahrens durchführbaren EMS-Training mit einer Flüssigkeit genässt und/oder einem Gel bestrichen wird, in der bzw. in dem Teilchen enthalten, insbesondere gelöst sind, welche als die Opferanode wirken und dazu aus dem Material mit negativem Standardelektrodenpotential in der elektrochemischen Spannungsreihe bestehen oder ein solches aufweisen, insbesondere aus dem Magnesium oder aus Magnesiumlegierungen, welches frei von Buntmetall wie z.B. Nickel ist.

## Claims

1. An EMS garment (2), for example, a suit, jacket, belt, shirt, trousers, armband, and/or legband, which carries a first number of EMS electrodes (1), wherein the first number of EMS electrodes (1) for transmitting EMS stimuli, which consists of current pulses and/or alternating current having predefined value such as amplitude and frequency, from a connected EMS stimulus generating unit (3) to the living body are each designed as a planar pad applied flexibly to the body or as a an electrically conducting conductive textile layer containing metallic or metallized threads, **characterized in that** the EMS garment (2) has at least one sacrificial anode (6), which is electrically conductively connected to the respective EMS electrode (1), wherein the sacrificial anode (6) consists of a material having a negative standard electrode potential in the electrochemical electromotive series or comprises such a material, wherein
the at least one EMS electrode (1) supporting a sacrificial anode (6) has a crimp plug, snap (5), or press connection bushing, on which the conductive layer is electrically conductively pressed, welded thereto, or sewn thereto, and/or on which an EMS signal line is clamped, soldered, sewn, or plugged on or in using a matching plug, wherein the sacrificial anode (6) is formed as a metal plate or strip attached to the crimp plug, snap (5), or the press connection bushing.

2. The EMS garment (2) according to claim 1, **characterized in that** the EMS garment (2) has at least one EMS electrode (1) supporting a sacrificial anode (6), which is electrically conductively connected or connectable to the conductive layer of the EMS electrode (1).

3. The EMS garment (2) according to claim 1 or 2, **characterized in that** the at least one EMS electrode (1) supporting a sacrificial anode (6) is a sewn-on EMS electrode (1) and/or an EMS electrode incorporated in a textile planar production method, for example, knitting, crocheting, weaving, or nonwoven material production.

4. An EMS garment (2), for example, a suit, jacket, belt, shirt, trousers, armband, and/or legband, which carries a first number of EMS electrodes (1), wherein the first number of EMS electrodes (1) for transmitting EMS stimuli, which consists of current pulses and/or alternating current having predefined value such as amplitude and frequency, from a connected EMS stimulus generating unit (3) to the living body are each designed as a planar pad applied flexibly to the body or as a an electrically conducting conductive textile layer containing metallic or metallized threads, **characterized in that** the EMS garment (2) has at least one sacrificial anode (6), which is electrically conductively connected to the respective EMS electrode (1), wherein the sacrificial anode (6) consists of a material having a negative standard electrode potential in the electrochemical electromotive series or comprises such a material, wherein
the EMS garment has a second number of electrical terminals for the first number of EMS electrodes, which second number of electrical terminals are each designed as a crimp plug, snap, or press connection bushing, and which are connected, via EMS signal lines pressed therein or sewn, soldered, or welded thereto, to the first number of EMS electrodes, wherein EMS signal cables for connection to the EMS stimulus generating unit are connectable to the second number of terminals, wherein a sacrificial anode is electrically conductively connected to each of the terminals.

5. The EMS garment according to claim 4, **characterized in that** the EMS signal lines which connect the second number of electrical terminals and the first number of EMS electrodes are designed as conductor tracks which are integrated, for example, knitted or woven, into the EMS garment.

6. The EMS garment according to claim 1 or 4, **characterized in that** the sacrificial anode is formed as a metal plate or strip and is adhesively bonded on the snap of the EMS electrode or the terminal.

7. The EMS garment according to claim 2 or 3, **characterized in that** the sacrificial anodes are each mounted by textile on the terminals, for example, embroidered or sewn on.

8. The EMS garment according to any one of the preceding claims, **characterized in that** the material having a negative standard electrode potential in the electrochemical electromotive series, of which the sacrificial anode (6) consists or which the sacrificial anode (6) comprises, is magnesium or a magnesium alloy, wherein the sacrificial anode (6) is free of nonferrous metal, for example, nickel.

9. An EMS exercise device (10), having a third number of EMS electrodes (1) attachable to the living body, for applying EMS stimuli to the body,
a fourth number of EMS stimulus generating units (3) electrically conductively connected to the third number of EMS electrodes (1), which units form a plurality of EMS stimuli, which follow a predefined excitation scheme and are distributed chronologically and with respect to the electrodes, from a current acquired from a current source, which stimuli consist of current pulses and/or alternating current having predefined value such as amplitude and frequency, and which are applied to the third number of EMS electrodes (1), to conduct current having predefined amplitude and frequency pattern through the body,
**characterized in that**
the EMS exercise device (10) has at least one sacrificial anode (6), which is electrically conductively connected to the EMS electrode (1), to protect the third number of EMS electrodes (1) and/or other elements in the electrically conductive connections from corrosion, wherein
each sacrificial anode (6) consists of a material having a negative standard electrode potential in the electrochemical electromotive series or comprises such a material, wherein
the EMS exercise device (10) has at least one EMS garment (2) according to any one of claims 1 to 8.

10. A method for ensuring the functionality of EMS electrodes or EMS garments according to claim 1 to 8 used in an EMS exercise device according to claim 9 over a long period of time, **characterized in that** a sacrificial anode is introduced on one or both sides of the body, **in that** at least one of the EMS electrodes is wetted using a liquid and/or coated using a gel before the EMS exercise which can be carried out after completion of the method, in which particles are contained, in particular dissolved, which act as the sacrificial anode and for this purpose consist of a material, preferably a metal, having a negative standard electrode potential in the electrochemical electromotive series or comprise such a material, in particular magnesium or magnesium alloys, which is free of nonferrous metal, for example, nickel.

## Revendications

1. Vêtement EMS (2), tel qu'un costume, une veste, une ceinture, une chemise, un pantalon, une bande de bras et/ou de jambe, qui porte un premier nombre d'électrodes EMS (1), dans lequel le premier nombre d'électrodes EMS (1) destinées à transmettre des stimuli EMS constitués d'impulsions de courant et/ou de courant alternatif avec des valeurs spécifiées telles que l'amplitude et la fréquence, d'une unité de génération de stimuli EMS connectée (3) au corps vivant, sont conçues chacune en tant que pad plat qui s'applique de manière flexible contre le corps ou en tant que section de structure textile ayant une couche conductrice textile électriquement conductrice et contenant des fils métalliques ou métallisés, **caractérisé par le fait que** le vêtement EMS (2) présente au moins une anode sacrificielle (6) qui est connectée de manière électriquement conductrice à l'électrode EMS (1) respective, dans lequel l'anode sacrificielle (6) consiste en un matériau ayant un potentiel d'électrode standard négatif dans la série de tension électrochimique ou présente un tel matériau, dans lequel ladite au moins une électrode EMS (1) portant une anode sacrificielle (6) présente une fiche mâle crimp, un bouton poussoir (5) ou une douille de connexion à pression, sur lequel ou bien laquelle la couche conductrice est pressée de manière électriquement conductrice, est soudée ou cousue, et/ou sur lequel ou bien laquelle une ligne de signal EMS est serrée, brasée, cousue ou rapportée ou bien enfichée au moyen d'une fiche mâle appropriée, dans lequel l'anode sacrificielle (6) est formée en tant que plaquette ou ruban métallique fixé(e) à la fiche mâle crimp, au bouton poussoir (5) ou à la douille de connexion à pression.

2. Vêtement EMS (2) selon la revendication 1, **caractérisé par le fait que** le vêtement EMS (2) présente au moins une électrode EMS (1) qui porte une anode sacrificielle (6) et qui est connectée ou peut être connectée de manière électriquement conductrice à la couche conductrice de l'électrode EMS (1).

3. Vêtement EMS (2) selon la revendication 1 ou 2, **caractérisé par le fait que** ladite au moins une électrode EMS (1) qui porte une anode sacrificielle (6) est une électrode EMS (1) cousue et/ou une électrode EMS qui est intégrée lors d'un procédé de fabrication de surface textile tel que, par exemple, le tricotage, le tissage, la production de non-tissé.

4. Vêtement EMS (2), tel qu'un costume, une veste, une ceinture, une chemise, un pantalon, une bande de bras et/ou de jambe, qui porte un premier nombre d'électrodes EMS (1), dans lequel le premier nombre d'électrodes EMS (1) destinées à transmettre des stimuli EMS constitués d'impulsions de courant et/ou de courant alternatif avec des valeurs spécifiées telles que l'amplitude et la fréquence, d'une unité de génération de stimuli EMS connectée (3) au corps vivant, sont conçues chacune en tant que pad plat qui s'applique de manière flexible contre le corps ou en tant que section de structure textile ayant une couche conductrice textile électriquement conductrice et contenant des fils métalliques ou métallisés, **caractérisé par le fait que** le vêtement EMS (2) présente au moins une anode sacrificielle (6) qui est connectée de manière électriquement conductrice à l'électrode EMS (1) respective, dans lequel l'anode sacrificielle (6) consiste en un matériau ayant un potentiel d'électrode standard négatif dans la série de tension électrochimique ou présente un tel matériau, dans lequel le vêtement EMS présente un deuxième nombre de connexions électriques pour le premier nombre d'électrodes EMS, lequel deuxième nombre de connexions électriques sont conçues chacune en tant que fiche mâle crimp, bouton poussoir ou douille de connexion à pression, et qui sont connectés au premier nombre d'électrodes EMS par l'intermédiaire de lignes de signal EMS qui y sont pressées ou cousues, brasées ou soudées, dans lequel des câbles de signal EMS destinés à la connexion à l'unité de génération de stimuli EMS peuvent être connectés au deuxième nombre de connexions, dans lequel respectivement une anode sacrificielle est montée de manière électriquement conductrice sur les connexions.

5. Vêtement EMS selon la revendication 4, **caractérisé par le fait que** les lignes de signal EMS reliant le deuxième nombre de connexions électriques et le premier nombre d'électrodes EMS sont conçues en tant que pistes conductrices intégrées, par exemple tricotées ou tissées, dans le vêtement EMS.

6. Vêtement EMS selon la revendication 1 ou 4, **caractérisé par le fait que** l'anode sacrificielle est réalisée en tant que plaquette ou ruban métallique et est collée sur le bouton poussoir de l'électrode EMS ou de la connexion.

7. Vêtement EMS selon la revendication 2 ou 3, **caractérisé par le fait que** les anodes sacrificielles sont chacune montées de manière textile, par exemple brodées ou cousues, sur les connexions.

8. Vêtement EMS selon l'une quelconque des revendications précédentes, **caractérisé par le fait que** le matériau à potentiel d'électrode standard négatif dans la série de tension électrochimique, dont est constituée l'anode sacrificielle (6) ou que présente l'anode sacrificielle (6), est du magnésium ou un alliage de magnésium, dans lequel l'anode sacrificielle (6) est exempte de métaux lourds non-ferreux tels que le nickel.

9. Dispositif d'entraînement EMS (10), comprenant un troisième nombre d'électrodes EMS (1) qui peuvent être attachées au corps vivant, pour appliquer des stimuli EMS au corps,
un quatrième nombre d'unités de génération de stimuli EMS (3) qui sont connectées de manière électriquement conductrice au troisième nombre d'électrodes EMS (1) et qui forment, à partir d'un courant obtenu d'une source de courant, une pluralité de stimuli EMS qui suivent un schéma d'excitation prédéfini et sont répartis dans le temps et par électrode et qui sont constitués par des impulsions de courant et/ou du courant alternatif présentant des valeurs prédéfinies telles que l'amplitude et la fréquence, et auxquelles le troisième nombre d'électrodes EMS (1) est soumis afin de faire passer du courant avec une amplitude et un motif de fréquence prédéterminés à travers le corps,
**caractérisé par le fait que**
le dispositif d'entraînement EMS (10) comprend au moins une anode sacrificielle (6) qui est connectée de manière électriquement conductrice à l'électrode EMS (1) afin de protéger de corrosion le troisième nombre d'électrodes EMS (1) et/ou d'autres éléments dans les connexions électriquement conductrices, dans lequel
chaque anode sacrificielle (6) consiste en ou comprend un matériau avec un potentiel d'électrode standard négatif dans la série de tension électrochimique, dans lequel
le dispositif d'entraînement EMS (10) comprend au moins un vêtement EMS (2) selon l'une quelconque des revendications 1 à 8.

10. Procédé destiné à assurer la fonctionnalité d'électrodes EMS ou bien de vêtements EMS selon la revendication 1 à 8, utilisés dans un dispositif d'entraînement EMS selon la revendication 9, sur une longue durée, **caractérisé en ce qu'**une anode sacrificielle est insérée d'un côté ou des deux côtés du corps par le fait qu'
au moins une des électrodes EMS est mouillée d'un liquide et/ou est enduite d'un gel avant l'entraînement EMS qui peut être fait après avoir terminé le procédé, le liquide ou bien le gel contenant des particules, en particulier sous forme dissolue, qui font fonction d'anode sacrificielle et, en plus, se composent du matériau ayant un potentiel d'électrode standard négatif dans la série de tension électrochimique ou présentent un tel matériau, en particulier du magnésium ou des alliages de magnésium, qui est exempt de métaux lourds non-ferreux tels que, par exemple, le nickel.
